# EUROPEAN PATENT APPLICATION

(11) **EP 2 143 787 A1**
(43) Date of publication of application: **13.01.2010**
(21) Application number: 08012596.6
(22) Date of filing: 11.07.2008
(51) Int. Cl.: C12N 5/00, C07H 19/00

(54) **A method for the formation of megamitochondria**

(71) Applicant: Universität Leipzig, 04109 Leipzig (DE)
(72) Inventor: Seibel, Peter, 97292 Uettingen (DE)
(74) Representative: Schüssler, Andrea

(57) **Abstract**

The present invention relates to an in-vitro method for the formation of megamitochondria in cells, wherein the cells are grown in a suitable fermentation medium acidulated with lactic acid to pH values between 5.3 and 6.7. The invention further concerns H⁺ ionophores, ionophores which catalyze the electroneutral exchange of K⁺ for H⁺ and inhibitors of actin polymerisation for the prevention or treatment of a disease in which inhibiting or reducing the formation of megamitochondria has a beneficial effect.

SEQUENCE LISTING

<110> Universität Leipzig

<120> A method for the formation of megamitochondria

<130> U 1068EP

<140> EP08012596
<141> 2008-07-11

<160> 4

<170> PatentIn version 3.3

<210> 1
<211> 27
<212> DNA
<213> artificial sequence

<220>
<223> primer

<400> 1
ggtgcagaat ggacctatag gacatcg 27

<210> 2
<211> 24
<212> DNA
<213> artificial sequence

<220>
<223> primer

<400> 2
tcaaagttgt tgccggtatc tcat 24

<210> 3
<211> 26
<212> DNA
<213> artificial sequence

<220>
<223> primer

<400> 3
agatctcatc gaggtgaagg ggaccc 26

<210> 4
<211> 30
<212> DNA
<213> artificial sequence

<220>
<223> primer

<400> 4
gaattctcaa agttgttgcc ggtatctcat 30

## Description

The present invention relates to an in-vitro method for the formation of megamitochondria in cells, wherein the cells are grown in a suitable fermentation medium acidified with lactic acid to pH values between 5.3 and 6.7. The invention further concerns H⁺ ionophores, ionophores which catalyze the electroneutral exchange of K⁺ for H⁺ and inhibitors of actin polymerisation for the prevention or treatment of a disease in which inhibiting or reducing the formation of megamitochondria has a beneficial effect.

In the literature mitochondria with highly exceeding diameters are known as megamitochondria. Physiological megamitochondria occur in many sperm cells (Hales und Fuller, 1997), in cold-blooded animal during hibernation (Grodums, 1977) and in the retina of many mammals (Samorajski *et al*., 1966; Kuhne, 1983; Lluch *et al*., 2003). Furthermore, they can be induced by chemicals like hydrazin or H₂O₂ (Karbowski *et al*., 1997, Karbowski *et al*., 1999) and they are accessory attributes of many human pathogenic disorders like alcohol liver (Rubin und Lieber, 1968; Iseri und Gottlieb, 1971; Yokoo *et al*., 1978; Chedid *et al*., 1980) and also in disorders caused by mutations of the mitochondrial DNA like MELAS (Pavlakis et al., 1984). This mutation has been associated with an A→G transition at position 3243 within the mitochondrial tRNA^{Leu(UUR)} gene and not only alters the tRNA^{Leu(UUR)} structure but also lies within a segment responsible for transcription termination of the rRNA genes. Besides defects like short stature, seizures, dementia, hearing loss and neurological presentations resembling strokes, on the cellular level a formation of megamitochondria is accompanied by lactic acidosis especially after exercise, distorted cristae structures and dysfunctions of the respiratory chain (Hirano et al., 1994; Goto et al., 1992; Ciafaloni et al., 1992).

These latter pathological attributes on cellular level are especial characteristics of culture cells depleted of mitochondrial DNA. These so called ρ⁰ cells proliferate without an intact oxidative phosphorylation where thirteen of the subunits composing respiratory chain complexes are absent. The energy production in ρ⁰ cells relies exclusively on an anaerobic glycolytic pathway. Furthermore, the cells are dependent on additional supplementation with pyruvate (King and Attardi, 1989) and uridine (Gregoire et al., 1984; Morais et al., 1980) and show a fast acidification of the culture medium due to excessive lactic acid fermentation (Jakobs et al., 1994). Structural investigations of mammalian ρ⁰ cells showed a disrupted network structure with small individual mitochondrial units distributed in the cytoplasm. The matrix seemed electron empty and most cristae showed a curved appearance lying in the matrix as concentric rings consisting of two membranes in close contact (Gilkerson *et al*., 2000).

Thus, the technical problem underlying the present invention is to provide a method for the formation of megamitochondria. These megamitochondria may be used as a tool to investigate mitochondrial diseases, like e.g. MELAS or mitochondrial myopathy, and to identify compounds for the treatment of diseases associated with the formation of megamitochondria or characterized by the presence of megamitochondria.

The solution to said technical problem is achieved by providing the embodiments characterized in the claims.

In studies leading to the present invention the formation of megamitochondria in an osteosarcoma cell line depleted of mitochondrial DNA (ρ⁰) was examined. It was found that the development of megamitochondria is dependent on both lactate and protons produced in the lactic acid fermentation and is associated with sizeable changes in cristae-structures. However, this is not due to apoptotic processes because upon the exchange of the acidic culture medium a rapid back-formation of megamitochondria to a network-like structure occurs. Furthermore, treatment of wild type osteosarcoma and Hela cells with lactic acid induces megamitochondria in cells still possessing mtDNA. These results suggest a lactic acid induced imbalance in fusion (= formation) and fission (= back-formation) that also indicates a mechanism that lead to the megamitochondria found in muscle biopsies of MELAS patients. Furthermore, the investigation of the influence of cytoskeleton components reveals for the first time a strong influence of the actin cytoskeleton on fusion and fission processes of mitochondria in mammalian cells. These results also indicate a connection between the observation of high lactic acid concentrations and the development of pathological megamitochondria as it can be observed in ultra structural investigation of muscle fibres of MELAS patients.

Finally, with the drugs CCCP, nigericin and valinomycin the influence of different components of the mitochondrial electrochemical membrane potential on the formation and back-formation of megamitochondria was examined. It was found that some of these ionophores (the H+ ionophore and the ionophore which catalyzes the electroneutral exchange of K+ for H+) can inhibit the formation of megamitochondria.

Accordingly, the present invention relates to an in-vitro method for the formation of megamitochondria in cells wherein the cells are grown in a suitable fermentation medium acidified with lactic acid to pH values between 5.3 and 6.7.

Suitable fermentation media are known to a person skilled in the art and depend from the cells to be grown. Examples are: Isocove medium, RPMI medium, Dulbecco's MEM medium, MEM medium, F12 Medium, Dulbecco's modified Eagle's medium or Minimum Essential Medium Eagle. These media may be supplemented with common supplements, like fetal calf serum, bromdeoxyuridine, pyruvate, uridine, Earle's salts, L-glutamine, sodium bicarbonate or non-essential amino acids.

The lactic acid is added to the fermentation medium until a pH between 5.3 and 6.7, preferably between 5.7 and 6.3, is reached. The concentration of lactic acid (i.e. lactate) in the medium is preferably 5 to 300 mM, more preferably 40-70 mM.

It is one of the characteristics of the method of the present invention that the formed megamitochondria are reversible by exchanging the fermentation medium which was acidified with lactic acid by a common fermentation medium having pH greater than 7.0, preferably equal or greater than pH 7.4.

Accordingly, the present invention further relates to an H⁺ ionophore and/or an ionophore which catalyzes the electroneutral exchange of K⁺ for H⁺ for the prevention or treatment of a disease in which inhibiting or reducing the formation of megamitochondria has a beneficial effect.

The present invention also relates to an inhibitor of actin polymerisation for the prevention or treatment of a disease in which inhibiting or reducing the formation of megamitochondria has a beneficial effect.

The term "megamitochondria" as used herein means mitochondria of a size that becomes visible in simple light microscopy, i.e. approximately >2 µm in diameter. Megamitochondria can be observed in different forms and shapes (spheres to needles).

Ionophores useful in the present invention are known to the person skilled in the art. Examples of suitable H⁺ ionophores comprise CCCP, FCCP or dinitrophenol. An examples of a suitable ionophore which catalyzes the electroneutral exchange of K⁺ for H⁺ is nigericin.

Inhibitors of actin polymerisation useful in the present invention are also known to the person skilled in the art. An examples of a suitable inhibitor is cytochalasin B.

The above compounds are useful for the prevention or treatment of any disease characterized by the presence or formation of megamitochondria or any disease in which the inhibition of the formation of megamitochondria or the promotion of megamitochondria back-formation has a beneficial effect. Examples of such diseases are mitochondrial myopathy, lactic acidosis, or MELAS.

For administration, the ionophore or inhibitor as described above is preferably combined with a suitable pharmaceutical carrier. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the ionophore or inhibitor as described above, preferably in combination with a pharmaceutically aceptable carrier, may be effected by different ways, e.g. by oral, intravenous, intraperetoneal, subcutaneous, intramuscular, topical or intradermal administration. The route of administration, of course, depends on the nature of the disease to be treated. The dosage regimen will be determined by the attending physician and other clinical factors. As is well known in the medical arts, dosages for any one patient depends on many factors, including the patient's size, body surface area, age, sex, time and route of administration, the kind of the disease, general health and other drugs being administered concurrently.

### BRIEF DESCRIPTION OF THE DRAWINGS

Figure 1
   **(A)** Co-localization analysis of the spherical aggregates in phase contrast with mitochondria by expression of EGFP-Mito fluorescence in 143B.TK⁻K7 cells
      Calibration marks correspond to 50 µm.
   **(B)** *In vivo* confocal microscopic documentation of the megamitochondria formation in 143B.TK⁻K7gm cells during the cultivation of 24 (A1), 40 (A2) 48 (A3) and 62 hours (A4)
      Mitochondrial staining was performed by stable expression of EGFP-Mito. Calibration marks correspond to 10 µm.
   **(C)** Graphical documentation of increase in diameter of megamitochondria during 40, 48 and 62 hours cultivation time
      The diameters of 100 mitochondria from different cells were examined by inverse confocal microscopy and analysed statistically. Error bars are indicated.
   **(D)** Analyses of megamitochondria formation by electron microscopy in 143B.TK⁻K7 cells 24 (A1-A2), 48 (B1-B2) and 62 (C1-C3) hours after seeding
      Images A1, B1 and C1 show overview sights. Details of single megamitochondria or internal structures respectively are depicted in A2, B2, C2 and C3. Calibration marks correspond to 1 µm.
Figure 2: *In vivo* confocal microscopic observation of megamitochondria back formation in 143B.TK⁻K7gm cells after exchange of used acidic medium by fresh medium
   The documentation of EGFP-Mito stained cells was carried out for 140 minutes. Calibration marks correspond to 10 µm.
Figure 3: Induction of megamitochondria by
   **(A)** incubation in acidic culture medium produced by 143B.TK⁻K7 cells after 68 hours of cultivation; or
   **(B)** by medium acidulated with lactic acid, pH 6.0-6.3, in the cell lines 143B.TK⁻K7 (A1-A3), 143B.TK⁻gm (B1-B3) and HeLa (C1-C3).
      Mitochondrial staining was performed as indicated. One hour after induction of megamitochondria the medium was exchanged by fresh medium to demonstrate the back-formation of megamitochondria (A2, B2, C2). Images A3, B3 and C3 depict untreated controls.

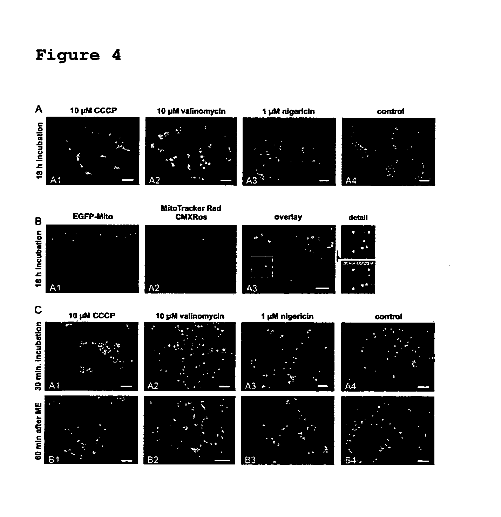

Figure 4: Influence of the mitochondrial membrane potential on the formation (A,B) and back-formation (C) of megamitochondria
   **(A)** 24 h after seeding 143B.TK⁻K7gm cells were incubated with 10 µM CCCP (A A1,), 10 µM valinomycin (A A2, B) or 1 µM nigericin (A A3) for 18 h.
   **(B)** Valinomycin-induced structures were analysed in detail (A3) by co-staining with EGFP-Mito (4B A1) and MitoTracker Red CMXRos (4B A2) that specifically stains the inner mitochondrial membrane (data not shown). Septating inner membranes stained by MitoTracker Red CMXRos or visible in phase contrast are indicated with white arrowheads.
   **(C)** 143B.TK⁻K7gm cells with megamitochondria were incubated with 10 µM CCCP (C A1, B1), 10 µM valinomycin (C A2, B2) or 1 µM nigericin (C A3, B3) for 30 minutes. 60 Minutes after medium exchange (ME) the mitochondrial morphology was analysed.
      Untreated controls are shown in A A4 and C A4, B4. Calibration marks correspond to 10 µm.

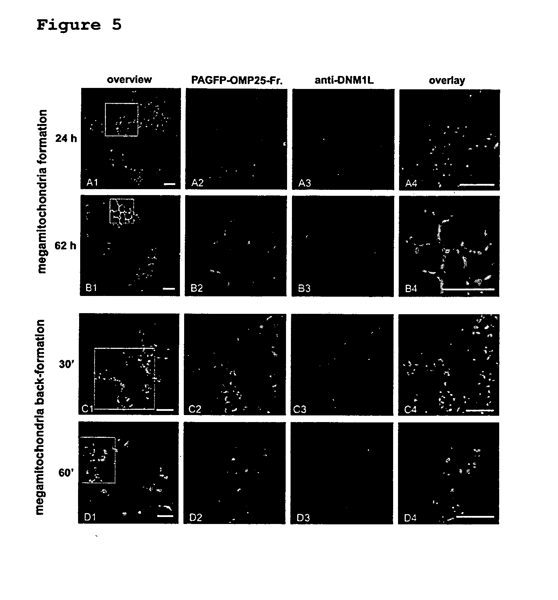

Figure 5: Confocal microscopic documentation of the endogenous localization of Drp1 during the development and back-formation of megamitochondria in 143B.TK⁻K7 cells
   Immunolocalization studies for the distribution of Drp1 during the formation of megamitochondria were done 24 (A1-A4) and 62 hours (B1-B4) of cultivation time and for the back-formation 30 (C1-C4) and 60 minutes (D1-D4) after exchange of acidic medium.
   Mitochondria were stained by expression of the outer mitochondrial membrane marker protein PAGFP-OMP25-Fr. (A2, B2, C2, D2). Secondary antibody of Drp1 detection was coupled to Cy3 (A3, B3, C3, D3). Images A1, B1, C1, D1 show overviews, detailed overlays are depicted in A4, B4, C4, D4. Calibration bars correspond to 5 µm.

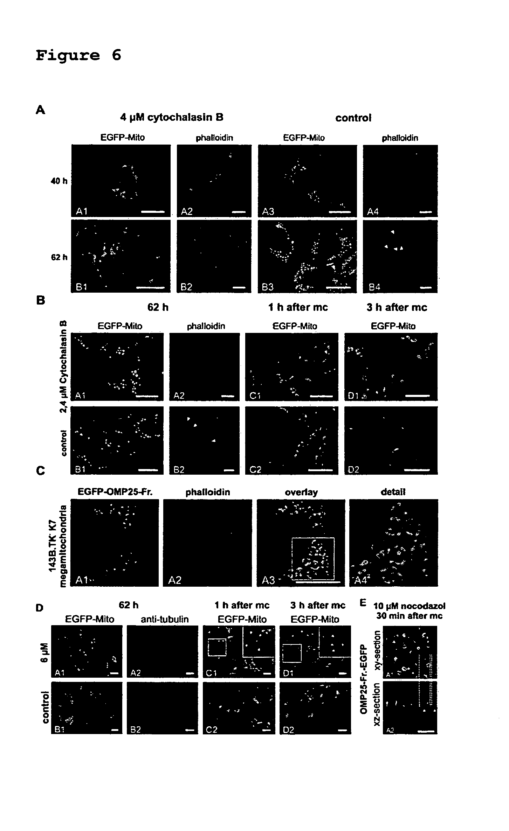

Figure 6: Influence of cytoskeleton components on the formation and back formation of megamitochondria
   **(A) - (C)** Influence of actin cytoskeleton on formation **(A)** and back-formation **(B, C)** of megamitochondria.
   **(A)** 143B.TK⁻K7gm cells stained with EGFP-Mito were treated with 4 µM cytochalasin B (A1, A2, B1, B2) at different cultivation times for 9 - 15 hours or an equivalent amount of solvent as control (A3, A4, B3, B4). Staining of actin cytoskeleton was achieved by Alexa Fluor 465 phalloidin in fixed cells (A2, B2, A4, B4). Arrow heads indicate round areas in the cytoplasm observable by actin staining after 62 hours cultivation time.
   **(B)** The influence of the actin cytoskeleton on the back-formation of megamitochondria in 143B.TK⁻K7gm cells was tested by treatment with 2.4 µM cytochalasin B (A1, A2, C1, D1) or an an equivalent amount of solvent as control (B1, B2, C2, D2) for 3 hours. Mitochondrial morphology was analyzed one (C1, C2) or three hours (D1, D2) after medium exchange. Staining of action cytoskeleton was carried out by Alexa Fluor 465 phalloidin in fixed cells (A2, B2).
   **(C)** Co-localization of ring-shaped actin-structures with megamitochondria of 143B.TK⁻K7 cells was carried out by transfection with pEGFP-OMP25-Fr. (A1) and staining of the actin cytoskeleton with Alexa Fluor 465 phalloidin in fixed cells (A2).
      Calibration marks correspond to 10 µm.
   **(D)** Influence of tubulin cytoskeleton on back-formation of megamitochondria.
      143B.TK⁻K7gm cells with megamitochondria were incubated for 3 hours with 6 µM nocodazol (A1, A2, C1, D1) or as control with an equivalent amount solvent (B1, B2, C2, D2). The analysis of mitochondrial changes in structure were carried out 1 hour (C1, C2) or 3 hours (D1, D2) after the exchange of acidic medium. Arrow heads indicate abnormal ring shaped mitochondrial structures persisting for at least 3 hours. Calibration bars correspond to 10 µm.
   **(E)** Confocal microscopic analyses of the ring-shaped mitochondrial structures were done with megamitochondria stained by EGFP-OMP25-Fr. after nocodazol incubation as described in (D). Shown is a xy-section in (A1) as well as the identical detail as xz-section (A2). Calibration marks correspond to 5 µm.

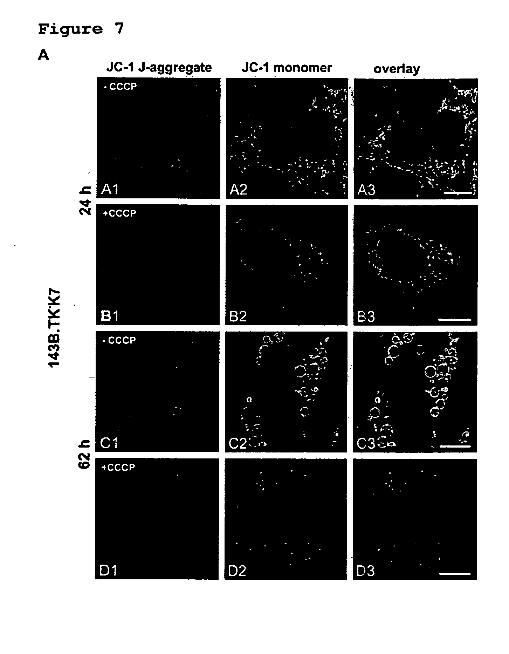

Figure 7: Documentation of mitochondrial membrane potential in living 143B.TK⁻K7.
   Documentation at 24 hours (A1-A3, B1-B3) and 62 hours (C1-C3, D1-D3) cultivation time by JC-1-staining. The red fluorescence of J-aggregates (A1, B1, C1, D1) as well as the green monomer form (A2, B2, C2, D2) were analysed simultaneously by excitation wavelength of 488 nm. Images A3, B3, C3, D3 show overlays. Calibration bars correspond to 10 µm.

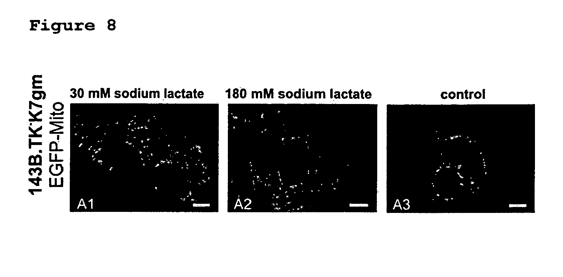

Figure 8: Induction of megamitochondria
   The induction of megamitochondria was not possible by incubation of 143B.TK⁻K7gm cells in acidified medium (HCl) or medium enriched with 30-180 mM sodium lactate without pH adjustment (pH 7.4 maintained) (A1, A2). Untreated controls are shown in A3. Calibration marks correspond to 10 µm.

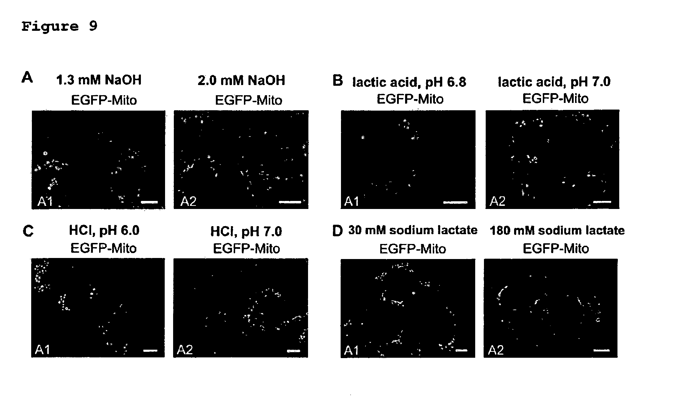

Figure 9: Influence of NaOH (A), lactic acid (B), HCl (C) and sodium lactate (D) to the back-formation of megamitochondria in 143B.TK⁻K7gm cells.
   A) The addition of 1.3 mM NaOH in the acidic medium (A1) did not lead to the back formation of megamitochondria. However, the addition of 2.0 mM NaOH was sufficient to induce the back-formation of megamitochondria (A2).
   B) Treatment of 143B.TK⁻K7gm cells with megamitochondria with fresh medium acidulated with lactic acid, pH 6.8 (B, A1) and pH 7.0 (B, A2) and C) HCl, pH 6.0 (C, A1) and pH 7.0 (C, A2) indicated a threshold value of megamitochondria back-formation depending on proton concentration.
   D) The persistence of megamitochondria is not dependent on lactate ions as shown in 143B.TK⁻K7gm cells with megamitochondria treated with medium completed with 30 mM (A1) or 180 mM sodium lactate (A2). Calibration marks correspond to 10 µm.

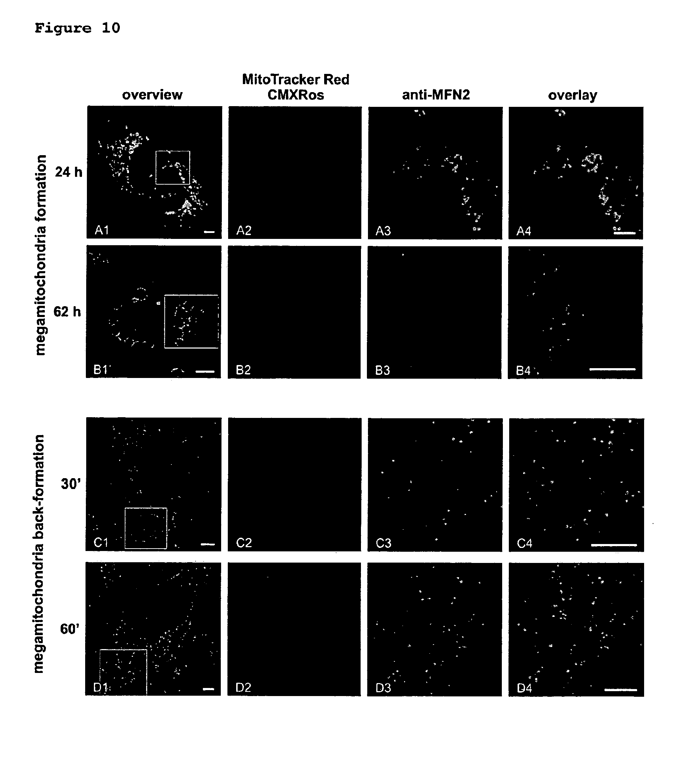

Figure 10: Confocal microscopic documentation of the endogenous localization of MFN2 during the development and back-formation of megamitochondria in 143B.TK⁻K7 cells.
   Immunolocalization studies for the distribution of MFN2 during the formation of megamitochondria were performed after 24 (A1-A4) and 62 hours (B1-B4) of cultivation time and for the back-formation 30 (C1-C4) and 60 minutes (D1-D4) after exchange of acidic medium. Mitochondria were stained with MitoTracker Red CMXRos (A2, B2, C2, D2). Secondary antibody of MFN2 detection was coupled to FITC (A3, B3, C3, D3). Images A1, B1, C1, D1 show overviews, detailed overlays are depicted in A4, B4, C4, D4. Calibration bars correspond to 5 µm.

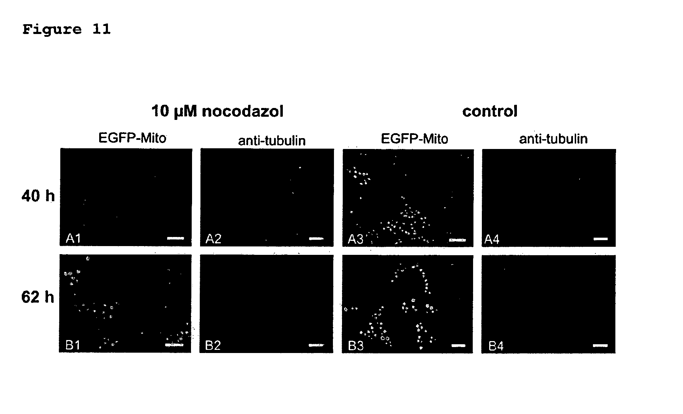

Figure 11: Influence of tubulin cytoskeleton on the formation of megamitochondria in 143B.TK⁻K7gm cells.
   143B.TK⁻K7gm cells were treated with 10 µM nocodazol at different cultivation times for 9 - 15 hours (A1, A2, B1, B2) or as control with an equivalent amount solvent (A3, A4, B3, B4). The morphology of mitochondria is shown by i*n vivo* EGFP-Mito-staining in the images A1, B1, C1, A3, B3, C3. The staining of tubulin cytoskeleton was performed by an antibody against alpha-tubulin and a Cy3-coupled secondary antibody in fixed cells (A2, B2, A4, B4). Calibration bars correspond to 10 µm.

The following Examples illustrate the invention.

### EXAMPLE 1

### Materials and Methods

### (A) Construction of mammalian expression plasmids

To generate the marker protein for outer mitochondrial membrane the C-terminal transmembrane region of OMP25 (Accession number NM_018373.1) which codes for the import and targeting signal to the mitochondrial outer membrane (Nemoto and De Camilli, 1999) was amplified from 143B.TK⁻ genomic DNA in a PCR with the following primers: hOMP25-303-FOR 5'-ggtgcagaatggacctataggacatcg-3' and hOMP-438-REV 5'-tcaaagttgttgccggtatctcat-3'. The amplificate was used as template in a PCR with the primers hOMP25-325-FOR 5'-agatctcatcgaggtgaaggggaccc-3' and hOMP-438-REV (2) 5'-paattctcaaagttgttgccggtatctcat-3' (*Bgl*II and *Eco*RI restriction sites underlined) and subcloned into pPAGFP-C1 and pEGFP-C1 with *Bgl*II and *Eco*RI.

### (B) Reagents and antibodies

Cytochalasin B and nigericin were obtained from Sigma-Aldrich (Taufkirchen, Germany); nocodazole, geneticin, CCCP, valinomycin, HCl and lacic acid were from AppliChem (Darmstadt, Germany) and MOWIOL from Calbiochem (Merck KGaA, Darmstadt, Germany).

Stock solutions in ethanol (1 mg/ml Cytochalasin B, 1 mM nigericin) or DMSO (10 mM Nocodazole, 10 mM CCCP, 10 mM valinomycin) were stored at -20°C.

MitoTracker Green FM, MitoTracker Red CMXRos, JC-1, secondary antibody Goat anti-Mouse IgG (H+L)-Cy3, secondary antibody Goat anti-Rabbit IgG (H+L)-FITC and Alexa Fluor 546 Phalloidin were obtained from Invitrogen (Karlsruhe, Germany). Primary polyclonal antibody against tubulin (K-alpha-1 (A02)) and primary monoclonal antibody against Drp1 were from Abnova Corporation (Taipei City, Taiwan), primary polyclonal antibody against MFN2 was a kind gift of Manuel Rojo, INSERM, U.582, University Pierre et Marie Curie, Paris, France

### (C) Cell culture

Human osteosarcoma cells 143B.TK⁻ wild type (ATCC c/o LGC Standards, Wesel, Germany) and 143B.TK⁻ ρ⁰ were cultured under standard conditions in Dulbecco's modified Eagle's medium (high glucose) with Glutamaxx (Invitrogen) supplemented with 5% fetal calf serum, 1% bromdeoxyuridine, 100 µg/ml pyruvate and 50 µg/ml uridine. HeLa cells were cultivated in Minimum Essential Medium Eagle with Earle's salts, L-glutamine and sodium bicarbonate (Sigma-Aldrich) supplemented with 5% fetal calf serum and 100 mM non-essential amino acids.

The transfection of 143B.TK⁻ and 143B.TK⁻ K7 cells with pEGFP-Mito (Clontech, Palo Alto, USA), pPAGFP-OMP25-Fr. and pEGFP-OMP25-Fr. were performed using Metafectene (Biontex, Martinsried, Germany). Selection with Geneticin (G418)(700 µg/ml) was carried out until the isolation of stable clones.

The induction of megamitochondria formation was done by the transfer of acidic medium from 143B.TK⁻ K7 ρ⁰ cells and by an artificial acidification of fresh medium with lactic acid, pH 6.0 - 6.3. Additional changes of culture medium were performed by an acidification of the medium with HCl, addition of 30 - 180 mM sodium lactate and 1.3 - 2.0 mM NaOH.

MitoTracker Green FM and MitoTracker Red CMXRos were used to label mitochondria according to manufacturer's protocol at a concentration of 400 nM.

Drugs (final concentrations 6 - 10 µM nocodazol, 2.4 - 4 µM cytochalasin B, 10 µM CCCP, 10 µM valinomycin and 1 µM nigericin) were added to standard culture medium at different stages of megamitochondria development.

### (D) Measurement of mitochondrial membrane potential

To measure differences in mitochondrial membrane potential between the various stages of megamitochondria formation and control cells the dual emission of the potentiometric dye JC-1 was observed. The cells either were incubated directly with 3 µM JC-1 diluted in the standard culture medium and filtered once with 0.2 µm syringe filter for 30 min at 37°C or first treated with CCCP (final concentration 10 µM) for 30 min for the comparison of cells without membrane potential. The cells were viewed *in vivo* with the inverse confocal laser scanning microscope (simultaneous excitation of red and green fluorescence by 488 nm wavelength) to observe local differences in the mitochondrial membrane potential.

### (E) Immunocytochemistry

Cells grown on cover slips were treated as indicated, fixed with methanol (-20°C) for 5 min and permeabilized with 0.2% Triton X-100 in PBS for 5 min at room temperature. After washing three times with PBS (5 min), the blocking reaction was performed with 100 mM glycine in PBS for 10 min following 3% BSA in PBS for 30 min at room temperature.

Subsequently cover slips were incubated with mouse anti-alpha tubulin polyclonal primary antibody diluted 1:50 in 1% BSA in PBS at 4°C over night. After washing the cells three times with PBS (5 min), the cover slips were incubated with Cy3-conjugated goat secondary antibody in 10% goat serum in PBS for 1 h at room temperature. The cells were washed three times with PBS (7 min) prior to mounting in Mowiol and analysed by fluorescence microscopy. For co-localization of Drp1 antibody and mitochondria 1 - 2 days after transfection of pOMP25-fragment-EGFP cells grown on cover slips were fixed with room temperature methanol/acetone (50%) for 2 min without the permeabilisation step and treated as above. Detection of endogenous MFN2 was carried out after methanol fixation (-20°C) for 5 min with the primary antibody against MFN2-NG diluted in 10% FCS for 30 min, subsequent washing steps in PBS and the secondary antibody Goat anti-Mouse IgG (H+L)-Cy3 in 10% goat serum in PBS for 1 h at room temperature.

For detection of the actin cytoskeleton cells on cover slips treated as indicated were fixed with 3.7% formaldehyde in PBS for 10 min at room temperature and after three washes in PBS for 5 min permeabilized with 0.1% Triton X-100 in PBS for 5 min. After additional washing steps with PBS (5 min) cells were blocked with 1% BSA in PBS for 30 min at room temperature and incubated with 1 unit (5 µl) Alexa Fluor 546 Phalloidin in 1% BSA in PBS for 20 min at room temperature. After washing the cells three times with PBS for 7 min, the cover slips were mounted with Mowiol and analysed with the confocal microscope.

### (F) Fluorescence Microscopy and Confocal Microscopy

Living cells were observed with the inverted fluorescence microscope DMI 6000 B (Leica Microsystems, Wetzlar, Germany) and cultured on glass bottom dishes (MatTek Corporation, Ashland, USA) with the inverted confocal laser scanning microscope TCS SP5 (Leica Microsystems, Wetzlar, Germany). To avoid a cross talk in excitation of multiple stained compounds always a sequential scanning mode was used in confocal microscopy.

Images were acquired with a charge-coupled device camera or with photo multipliers respectively and micrographs were processed and analyzed with the software Leica Application Suite Advanced Fluorescence 1.5.1 and Adobe Photoshop CS (Version 8.0.1).

### (G) Electron microscopy

Cells grown on cover slips were fixed with a solution of 2.5% glutaraldehyde, 2% formaldehyde (made from paraformaldehyde) in 100 mM cacodylate buffer pH 7.4 for 1.5 h at 4°C, washed twice with cacodylate buffer, dehydrated with ethanol followed by propylene oxide, and immersed in a mixture of propylene oxide and epon 1:1 for 1 - 2 h. Finally, the cells were embedded in epon by polymerization at 60°C for 2 days. Ultrathin sections were analyzed with a Zeiss EM10 (Zeiss/Oberkochen, Germany).

### EXAMPLE 2

### Formation of megamitochondria in osteosarcoma ρ⁰ cell lines

Since the development of the first ρ⁰ culture cells (Desjardins et al., 1985; Desjardins et al., 1986; King and Attardi, 1988; Morais et al., 1988) many investigations were carried out to use this model system for gaining insights into mitochondrial-nuclear interactions. This study focused on properties of these cells that resemble pathological features that can be observed in disorders connected to mitochondrial DNA mutations.

The ρ⁰ cells used in that study were derived from the osteosarcoma cell line 143B.TK⁻ by the incubation with low doses of ethidium bromide (143B.TK⁻ρ⁰) (King and Attardi, 1988) or by the transient expression of a mitochondrially targeted restriction endonuclease (143B.TK⁻K7). Identical experiments were carried out for the parental cell line (143B.TK⁻; wild type; cells containing mitochondrial DNA). The formation of megamitochondria described for the rho⁰ cell line was found to be the same in the parental cell line.

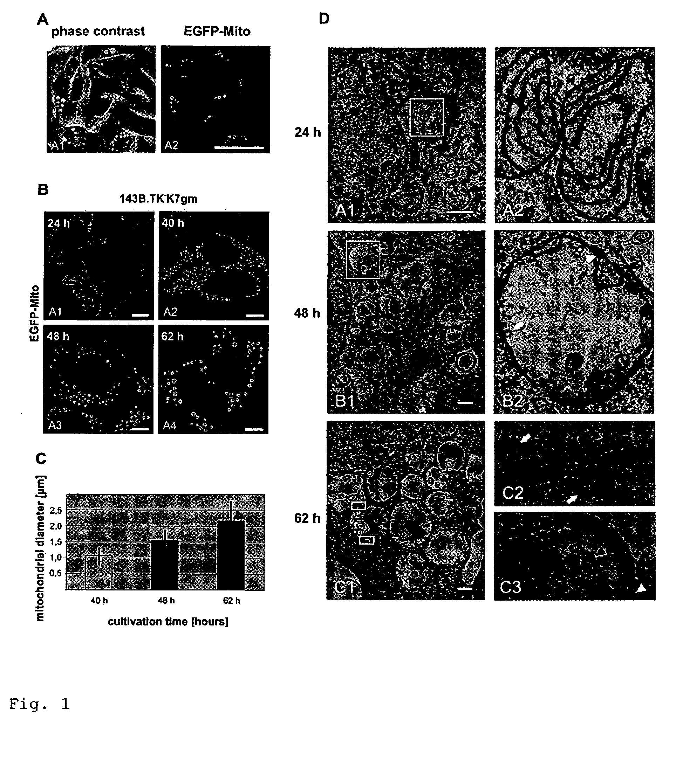

During the cultivation of these ρ⁰ cell lines spherical, low-contrasted aggregates that persistently increased occurred in the cytoplasm. A transient expression of EGFP-Mito revealed a colocalization of the spherical aggregates with the EGFP-Mitostaining indicating that the mitochondrial network structure has changed to morphology denoted as megamitochondria (Fig. 1A). The formation of megamitochondria in both ρ⁰ cell lines 143B.TK⁻ρ⁰ and 143B.TK⁻K7 that were developed by different methodical approaches indicates that this is a fundamental attribute of 143B.TK⁻ cells in a ρ⁰ state. To document this formation the cell line 143B.TK⁻K7 was stably transfected with pEGFP-Mito resulting in the cell line 143B.TK⁻K7gm. Analyses of the mitochondrial network were performed at 24, 40, 48 and 62 hours cultivation time by confocal microscopy (Fig. 1B). Additionally this increase of megamitochondria during the cultivation was evaluated statistically by defining the diameters of 100 mitochondria from different cells (Fig. 1C).

From a mitochondrial network characteristic for ⁰ cells with single mitochondrial units that seemed to be swollen after 24 h (Fig. 1B A1) with proceeding cultivation time initially small spherical mitochondria persisting of single units with a considerable larger diameter emerged (Fig. 1B A2, A3, A4). Concomitantly the overall number of mitochondria decreased up to 10 - 20 mitochondria per cell 62 hours after seeding (Fig 1B A4). This is due not only to an extreme swelling of the mitochondria but also numerous fusion events. The averages of mitochondrial diameters increased with continuing cultivation time from 1.08 ± 0.24 µm at 40 hours to 1.58 ± 0.29 µm after 48 hours and up to 2.17 ± 0.59 µm at 62 hour cultivation time (Fig 1C). With an optimal ratio of seeded cell number to medium volume and cultivation time it was possible to gain megamitochondria with diameters up to 7 µm.

In spite of the formation of megamitochondria the 143B.TK⁻K7gm cells proliferated continuously and characteristic apoptotic criteria like rounding of the cell, deformations of nucleus or generation of "*apoptotic bodies*" could not be observed. However an intensive acidification of the culture medium caused a rounding of the cells and they detached in large connected cell structures from the culture dish bottom yielding in cell death.

To document changes of mitochondrial cristae structures transmission electron microscopic examinations of megamitochondria formation were carried out at 24, 48 and 62 hours cultivation time (Fig. 1D). In early stages of the megamitochondria generation a mitochondrial appearance denoted as "*fuzzy onions*" with single mitochondrial units often with swollen appearance and electron empty matrix (Fig. 1D A1, A2) was visible. With an increase in mitochondrial diameter less inner membrane structures are discernable and the electron density further declined. Some-circular double membrane sections could be observed (Fig. 1D B2, C2, C3 black arrowheads) as well as semi circular compartments that still had connections with the inner mitochondrial border membrane (Fig. 1D B2, C3 white arrowheads). In spite of the excessive morphological changes the mitochondria still obtained a double membrane envelope. Additionally numerous inversions and inclusions consisting of one membrane in the matrix could be identified (Fig. 1D B2, C2 white arrows). Overall these changes present enormous changes of mitochondrial cristae structures associated with the formation of megamitochondria.

### EXAMPLE 3

### Back-formation of megamitochondria after exchange of the acidic culture medium

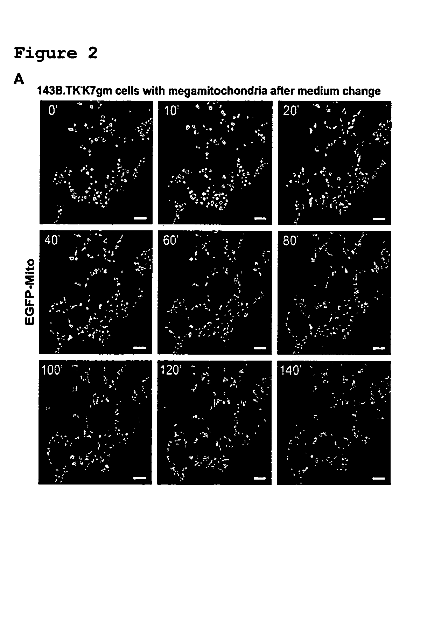

Upon exchanging the acidic culture medium with fresh medium the megamitochondria of 143B.TK⁻K7gm cells showed a rapid back-formation into a mitochondrial network that is typical for ρ⁰ cells. This was documented by confocal microscopy (Fig. 2). Ten minutes after adding fresh medium most of the mitochondria depicted an altered morphology with slight deformations. During the subsequent progress the megamitochondria exhibited an oval and elongated form and further tubulation occurred. Additionally branched mitochondria appeared and single punctiform mitochondria emanated.

Therefore, the back-formation of megamitochondria is a rapid process induced by the exchange of the acidic culture medium and is accompanied by mitochondrial fission events.

### EXAMPLE 4

### Examination of the mitochondrial membrane potential with the dye JC-1 during the megamitochondria formation

To determine the mitochondrial membrane potential during the formation of megamitochondria 143B.TK⁻K7 cells were either directly stained with JC-1 (3 µg/ml) or incubated with 10 mM CCCP for 30 minutes before. This was accomplished as comparison for mitochondria not possessing a membrane potential. The analyses of local differences in membrane potential of megamitochondria were performed after 24 until 62 hours cultivation time by confocal microscopy after 30 minutes of incubation with JC-1 (Fig.7). The mitochondria of the 143B.TK-K7 cells exhibited a membrane potential after 24 hours (Fig. 7 A1-A3) as well as after 62 hours (Fig. 7 C1-C3) of cultivation time. This was apparent by comparing the staining with CCCP-treated cells (Fig. 7 B1-B3, D1-D3). Here, the red fluorescence was clearly less distinct and the green monomer fluorescence displayed a diffuse distribution within the cells. Additionally the images show that the JC-1 dye stains a mitochondrial membrane surrounding the mitochondria.

### EXAMPLE 5

### Induction and analyses of the megamitochondria formation by changes of culture medium

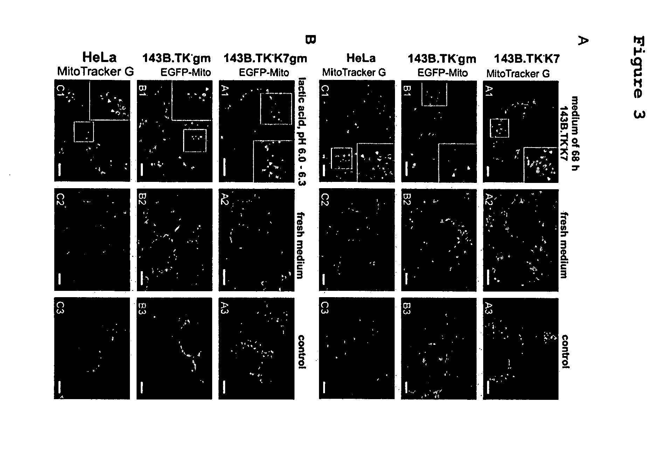

To gain insights which factors are involved in the formation of the megamitochondria the feasibility to induce the megamitochondria extrinsically was examined. First, the possibility that the megamitochondria formation is due to a starvation process of these cells was excluded by adding 4,5 g/l glucose in the acidic medium. However this restoration of nutrients did not lead to any changes in the structure of megamitochondria (data not shown). Therefore, 143B.TK⁻K7, 143B.TK⁻gm and HeLa cells were incubated with used acidic culture medium of 143B.TK⁻K7 cells with megamitochondria to test whether this medium contains substances that lead to the induction of megamitochondria (Fig. 3A). After one hour of incubation the mitochondrial morphology was analysed *in vivo* after staining the 143B.TK⁻K7 and HeLa cells with MitoTracker Green FM.

In all used cell lines the mitochondrial network changed from an expanded rod and tubule shape to numerous spherical mitochondria with differing diameter (Fig. 3B and Fig 3B A1, B1, C1 white arrowheads). However the diameter of these megamitochondria did not reach the size of intrinsic megamitochondria of ρ⁰ cells.

To test whether megamitochondria can be induced by extrinsic changes of pH value or lactic acid concentration in the culture medium 143B.TK⁻K7gm, 143B.TK⁻gm and HeLa were incubated with medium complemented with HCl and lactic acid, respectively (pH 6.7 - 5.3). Additionally, the influence of high concentrations of sodium lactate (30 - 180 mM) on the megamitochondria formation was determined without changing the pH. A pH value of 6.7 - 6.8 and a lactate concentration of 30 mM corresponds to the measured values in the analysed cultivation medium of 143B.TK⁻K7 cells. Incubation of cells was carried out for one hour with subsequent analyses of mitochondrial morphology *in vivo*. The induction of megamitochondria in all cell lines tested was not possible with culture medium acidified with HCl or after incubation with high sodium lactate concentrations without pH adjustment (pH 7.4 maintained)(Fig. 8).

However, the incubation of 143B.TK⁻K7gm, 143B.TK⁻gm and HeLa cells with medium artificially acidified with lactic acid to pH values 5.3 - 6.7 led to the formation of megamitochondria in these cell lines (Fig. 3B A1, B1, C1 white arrowheads) though they did not reach the dimensions of intrinsic ones.

To assure that these observed mitochondrial changes are reversible and not a sign of apoptosis the acidic medium was changed again and replaced by fresh medium. One hour after incubation the mitochondria were observed *in vivo*. In all treated cell lines the characteristic mitochondrial network formed back (Fig. 3A A2, B2, C2, 3B A2, B2, C2).

Therefore the megamitochondria can be induced by substances contained in the used culture medium of 143B.TK⁻K7 cells with megamitochondria. It is not sufficient to add only protons or lactate to the medium to induce the formation of megamitochondria but rather sufficient amounts of protons and lactate anions are essential. Furthermore, it could be evidenced that also cells still containing mtDNA develop megamitochondria in these conditions. However, their megamitochondria show a decreased diameter in contrast to the intrinsic ones in the ρ⁰ cells. Thus, the metabolic changes generating excessive amounts of lactate as well as protons due to the ρ⁰ state of the cells are responsible for the formation of megamitochondria in the 143B.TK⁻K7 and 143B.TK⁻ρ⁰ cells.

Similar experiments were carried out to investigate the back-formation of megamitochondria (Fig. 9). To this end, 143B.TK⁻K7gm cells with megamitochondria were incubated in medium complemented with different additions. Changes of pH value were reached by the addition of 0.04 - 0.2 mM NaOH into the used culture medium as well as by the addition of HCl and lactic acid, respectively, of values 7.0 - 5.6 in fresh culture medium. Moreover, the influence of high concentrations of sodium lactate on the back-formation of megamitochondria was analysed by adding 20 - 210 mM sodium lactate, pH 7.4 into fresh medium without changing the pH value. Treatment of cells with different media was performed for one hour, thereafter the mitochondrial morphology was analysed by fluorescence microscopy. The addition of NaOH into the acidic medium of 143B.TK⁻K7gm cells with megamitochondria reversed the pH value of the medium to alkaline. Addition of 1.3 mM NaOH did not lead to the back-formation of megamitochondria. However, the addition of 2.0 mM NaOH into the medium resulted in a change in mitochondrial morphology from spherical forms to tubules and network-like structures (Fig. 9, A). Fresh medium artifically acidificated with HCl exhibits a low pH value but no high lactate concentration. At pH 6.0 the megamitochondria did not change their form although at pH 7.0 the megamitochondria formed back to a tubular network (Fig. 9 C). The pH threshold value where a change from spherical to tubular structures occurred was not defined sharply but emerged in a continuing manner with also gradual morphologic changes. The addition of sodium lactate to fresh medium led to the back-formation of megamitochondria in all used concentrations (Fig. 9, D). This evidenced that the presence of high lactate concentrations did not avoid the back-formation of megamitochondria. Furthermore, medium acidulated with lactic acid at a pH value of 7.0 also resulted in the back-formation of megamitochondria in contrast to medium with pH 6.8 where the spherical mitochondria changed to a tubular network (Fig. 9, B).

Altogether, the results show that for the back-formation of mitochondria a threshold level of sufficient low proton concentration must be given in the medium. The existence of lactate anions in the culture medium is not essential for the persistence of megamitochondria.

### EXAMPLE 6

### Influence of the mitochondrial membrane potential on the formation and back-formation of megamitochondria

To investigate the influence of the mitochondrial membrane potential on the formation and back formation of the megamitochondria 143B.TK⁻K7gm cells were incubated with 10 µM CCCP (H⁺ ionophore,), 10 µM valinomycin (K⁺ selective ionophore) and 1 µM nigericin (selective electron neutral K⁺/H⁺ exchange) (Fig. 4).

The formation of megamitochondria in the CCCP and nigericin treated cells was inhibited. The network structure rather was highly fragmented and aggregated around the nucleus (Fig 4A A1, A3). In contrast, the incubation with valinomycin led to the formation of megamitochondria with diameters exceeding the control cells (Fig. 4A A2). Furthermore, the mitochondria seemed to be septated by unfused inner membranes as described in experiments of other groups (Malka *et al*., 2005). These structures were confirmed in detail (Fig. 4B) by co-staining with EGFP-Mito and MitoTracker Red CMXRos that specifically stains the inner mitochondrial membrane (data not shown).

To investigate the influence of the different chemicals on the back-formation of megamitochondria the cells were incubated with indicated concentrations of CCCP, valinomycin and nigericin for 30 minutes (Fig. 4C). After the exchange of the acidic medium by fresh medium also complemented by the different drugs, the mitochondrial morphology of the CCCP and nigericin treated cells was comparable to the control cells (Fig. 4C B1, B3, B4). However, valinomycin inhibited the back-formation of megamitochondria to a mitochondrial network but led to even larger megamitochondria with septae of unfused inner mitochondrial membranes (Fig. 4C B2).

In summary the experiments depict a severe influence of CCCP and nigericin on the formation but not back-formation of megamitochondria. A contrary influence could be observed by the incubation with valinomycin that induced even larger megamitochondria in conditions leading to the formation but also back-formation of megamitochondria.

### EXAMPLE 7

### Localization of fusion and fission proteins during the formation and back-formation of megamitochondria

The formation of megamitochondria by the acidification of culture medium is accompanied by the increase of mitochondrial diameters as well as by a decrease of the overall number of mitochondria per cell. Accordingly, the dynamic balance of fusion and fission is strongly shifted towards fusion. Conversely, fission events are induced by the exchange of used acidic culture medium by fresh medium. Hence, the native localization and distribution of the mitochondrial fusion protein MFN2 and fission protein Drp1 were examined by immunocytochemistry to gain insights in the involvement of these proteins in the megamitochondrial formation and back-formation processes. The analyses of MFN2 revealed a mitochondrial localisation during the formation as well as back-formation of megamitochondria. There, MFN2 was located in discrete punctiform domains in the outer mitochondrial membrane. Additionally no quantitative changes of MFN2 on the mitochondria could be observed (Fig. 10).

The localization of endogenous Drp1 also was investigated during the formation and back formation of megamitochondria (Fig. 5). The distribution of Drp1 throughout the formation of megamitochondria was unevenly located in punctuate structures of the outer mitochondrial membrane additional to foci in the cytoplasm (Fig. 5 A1-A4, B1-B4). During the back-formation the majority of punctiform Drp1 areas relocate from the cytoplasm to presumably fission sites on the mitochondria (Fig. 5 C1-C4, D1-D4).

These results show that both MFN2 and Drp1 depicted a mitochondrial localization in conditions favouring fusion as well as fission events. This indicates that the accomplishment of fission processes during the formation of megamitochondria presumably is inhibited.

### Example 8

### Influence of cytoskeletal components on formation and back-formation of megamitochondria

The shape of mitochondria in living cells undergoes a strong variation from single mitochondrial units to a widespread network. Additionally, the distribution of mitochondria within one cell often is heterogeneous with an accumulation in areas of high energy consumption. Hence, mitochondria display certain mobility with transport mechanisms involving cytoskeletal protein components.

To observe the influence of the tubulin or actin cytoskeleton, respectively, on the formation of megamitochondria 143B.TK⁻K7gm cells were incubated with 10 µM nocodazol or 4 mM cytochalasin B for 9 - 15 hours. The comparison of drug-treated cells with controls *in vivo* by fluorescence microscopy exhibited no influence of the tubulin cytoskeleton on the formation of megamitochondria (Fig. 11). Complete inhibition of tubulin polymerisation was evidenced by immunocytochemical staining with an antibody against alpha-tubulin. Incubation of cells with the inhibitor of actin polymerisation, cytochalasin B, prevented formation of megamitochondria, while confirming the effectiveness of drug treatment by phalloidin staining (Fig. 6A).

The influence of tubulin and actin cytoskeleton inhibition on back-formation of megamitochondria was performed with 143B.TK⁻K7gm cells with megamitochondria 3 hours after incubating the cells with 6 µM nocodazol or 2.4 mM cytochalasin B. After changing the used acidic medium and its replacement by fresh medium also completed by the drugs structural changes of mitochondria were observed one hour and three hours later.

The inhibition of tubulin cytoskeleton had only minor influence on back-formation of megamitochondria (Fig. 6D). The nocodazol-treated cells displayed a network like mitochondrial structure similar to the controls. However besides numerous single mitochondria also ring-shaped mitochondrial structures could be observed in confocal microscopy (Fig. 6D, white arrowheads). These structures were further investigated by transient transfection of 143B.TK⁻K7 with pEGFP-OMP25-Fr that stains the mitochondrial outer membrane. Comparing xz-sections along the denoted white line indicated cup-like mitochondria bent in one direction (Fig. 6E white arrowheads). Depending on the focus level this mitochondrial cup appeared as a ring in confocal microscopy sections.

The inhibitor of actin-polymerisation, cytochalasin B, had a severe influence on megamitochondria back-formation. Indeed deformations of the megamitochondria occurred but no proceeding tubulation and reconstitution of mitochondrial network could be observed (Fig. 6B). Additionally, the presented images indicate that the megamitochondria were embedded in rings of actin (Fig. 6B B2, white arrowheads) as it is confirmed by co-staining of actin by phalloidin and megamitochondria with EGFP-OMP25-Fr after transient transfection (Fig. 6C).

Recapitulatory, these results evidence no influence of the tubulin cytoskeleton on the formation of megamitochondria but an involvement in tubulation during back-formation. No enlarged mitochondria occurred during cytochalasin B-treatment and also the reconstitution of a mitochondrial network was disturbed after the exchange of used medium.

### LIST OF REFERENCES

Adachi, K., T.Matsuhashi, Y.Nishizawa, J.Usukura, M.Momota, J.Popinigis, and T.Wakabayashi. 1994. Further studies on physicochemical properties of mitochondrial membranes during the formation process of megamitochondria in the rat liver by hydrazine. Exp. Mol. Pathol. 61:134-151.
Adachi, K., M.Momota, Y.Teranishi, R.Ueki, M.Hagiwara, T.Wakabayashi, and J.Popinigis. 1992. Effects of alkyl alcohols and related chemicals on rat liver structure and function. III. Physiochemical properties of ethanol-, propanol- and butanol- treated rat liver mitochondrial membranes. Acta Pathol. Jpn. 42:549-557.
Chedid, A., W.Jao, and J.Port. 1980. Megamitochondria in hepatic and renal disease. Am. J. Gastroenterol. 73:319-324.
Ciafaloni, E., E.Ricci, S.Shanske, C.T.Moraes, G.Silvestri, M.Hirano, S.Simonetti, C.Angelini, M.A.Donati, C.Garcia, and . 1992. MELAS: clinical features, biochemistry, and molecular genetics. Ann. Neurol. 31:391-398.
Desjardins, P., J.M.de Muys, and R.Morais. 1986. An established avian fibroblast cell line without mitochondrial DNA. Somat Cell Mol Genet 12:133-9.
Desjardins, P., E.Frost, and R.Morais. 1985. Ethidium bromide-induced loss of mitochondrial DNA from primary chicken embryo fibroblasts. Mol Cell Biol 5:1163-9.
Gilkerson, R.W., D.H.Margineantu, R.A.Capaldi, and J.M.Selker. 2000. Mitochondrial DNA depletion causes morphological changes in the mitochondrial reticulum of cultured human cells. FEBS Lett. 474:1-4.
Goto, Y., S.Horai, T.Matsuoka, Y.Koga, K.Nihei, M.Kobayashi, and I.Nonaka. 1992. Mitochondrial myopathy, encephalopathy, lactic acidosis, and stroke-like episodes (MELAS): a correlative study of the clinical features and mitochondrial DNA mutation. Neurology 42:545-550.
Gregoire, M., R.Morais, M.A.Quilliam, and D.Gravel. 1984. On auxotrophy for pyrimidines of respiration-deficient chick embryo cells. Eur J Biochem 142:49-55.
Grodums, E.I. 1977. Ultrastructural changes in the mitochondria of brown adipose cells during the hibernation cycle of Citellus lateralis. Cell Tissue Res. 185:231-237.
Hales, K.G. and M.T.Fuller. 1997. Developmentally regulated mitochondrial fusion mediated by a conserved, novel, predicted GTPase. Cell. 90:121-129.
Hawley, E.S. and R.P.Wagner. 1967. Synchronous mitochondrial division in Neurospora crassa. J. Cell Biol. 35:489-499.
Hayashi, J., S.Ohta, Y.Kagawa, D.Takai, S.Miyabayashi, K.Tada, H.Fukushima, K.Inui, S.Okada, Y.Goto, and. 1994. Functional and morphological abnormalities of mitochondria in human cells containing mitochondrial DNA with pathogenic point mutations in tRNA genes. J. Biol. Chem. 269:19060-19066.
Hirano, M. and S.G.Pavlakis. 1994. Mitochondrial myopathy, encephalopathy, lactic acidosis, and strokelike episodes (MELAS): current concepts. J. Child Neurol. 9:4-13.
Iseri, O.A. and L.S.Gottlieb. 1971. Alcoholic hyalin and megamitochondria as separate and distinct entities in liver disease associated with alcoholism. Gastroenterology. 60:1027-1035.
Ishihara, N., A.Jofuku, Y.Eura, and K.Mihara. 2003. Regulation of mitochondrial morphology by membrane potential, and DRP1-dependent division and FZO1-dependent fusion reaction in mammalian cells. Biochem. Biophys. Res. Commun. 301:891-898.
Jakobs, B.S., B.C.van den, G.Dacremont, and R.J.Wanders. 1994. Beta-oxidation of fatty acids in cultured human skin fibroblasts devoid of the capacity for oxidative phosphorylation. Biochim. Biophys. Acta. 1211:37-43.
Karbowski, M., C.Kurono, Y.Nishizawa, Y.Horie, T.Soji, and T.Wakabayashi. 1997. Induction of megamitochondria by some chemicals inducing oxidative stress in primary cultured rat hepatocytes. Biochim. Biophys. Acta. 1349:242-250.
Karbowski, M., C.Kurono, M.Wozniak, M.Ostrowski, M.Teranishi, Y.Nishizawa, J.Usukura, T.Soji, and T.Wakabayashi. 1999a. Free radical-induced megamitochondria formation and apoptosis. Free Radic. Biol. Med. 26:396-409.
Karbowski, M., C.Kurono, M.Wozniak, M.Ostrowski, M.Teranishi, T.Soji, and T.Wakabayashi. 1999b. Cycloheximide and 4-OH-TEMPO suppress chloramphenicol-induced apoptosis in RL-34 cells via the suppression of the formation of megamitochondria. Biochim. Biophys. Acta. 1449:25-40.
King, M.P. and G.Attardi. 1988. Injection of mitochondria into human cells leads to a rapid replacement of the endogenous mitochondrial DNA. Cell. 52:811-819.
King, M.P. and G.Attardi. 1989. Human cells lacking mtDNA: repopulation with exogenous mitochondria by complementation. Science. 246:500-503.
Kuhne, J.H. 1983. Rod receptors in the retina of Tupaia belangeri. Anat. Embryol. (Berl). 167:95-102.
Kukat, A., C.Kukat, J.Brocher, I.Schafer, G.Krohne, I.A.Trounce, G.Villani, and P.Seibel. 2008. Generation of rhoO cells utilizing a mitochondrially targeted restriction endonuclease and comparative analyses. Nucleic Acids Res. 36:e44.
Legesse-Miller, A., R.H.Massol, and T.Kirchhausen. 2003. Constriction and Dnmlp recruitment are distinct processes in mitochondrial fission. Mol. Biol. Cell. 14:1953-1963.
Legros, F., A.Lombes, P.Frachon, and M.Rojo. 2002. Mitochondrial fusion in human cells is efficient, requires the inner membrane potential, and is mediated by mitofusins. Mol. Biol. Cell. 13:4343-4354.
Lluch, S., M.J.Lopez-Fuster, and J.Ventura. 2003. Giant mitochondria in the retina cone inner segments of shrews of genus Sorex (Insectivora, Soricidae). Anat. Rec. A Discov. Mol. Cell Evol. Biol. 272:484-490.
Malka, F., O.Guillery, C.Cifuentes-Diaz, E.Guillou, P.Belenguer, A.Lombes, and M.Rojo. 2005. Separate fusion of outer and inner mitochondrial membranes. EMBO Rep. 6:853-859.
Matsuhashi, T., X.Liu, M.Karbowski, M.Wozniak, J.Antosiewicz, and T.Wakabayashi. 1997. Role of free radicals in the mechanism of the hydrazine-induced formation of megamitochondria. Free Radic. Biol. Med. 23:285-293.
Matsuhashi, T., X.Liu, Y.Nishizawa, J.Usukura, M.Wozniak, and T.Wakabayashi. 1996. Mechanism of the formation of megamitochondria in the mouse liver induced by chloramphenicol. Toxicol. Lett. 86:47-54.
Mattenberger, Y., D.I.James, and J.C.Martinou. 2003. Fusion of mitochondria in mammalian cells is dependent on the mitochondrial inner membrane potential and independent of microtubules or actin. FEBS Lett. 538:53-59.
McGill, M. 1980. Inhibition of mitochondrial-specific protein symthesis in human lymphocytes and platelets is dependent upon the stage of cellular differentiation. Cytogenet. Cell Genet. 26:117-126.
Meeusen, S., J.M.McCaffery, and J.Nunnari. 2004. Mitochondrial fusion intermediates revealed in vitro. Science. 305:1747-1752.
Morais, R. 1996. Isolation of avian mitochondrial DNA-less cells. Methods Enzymol. 264:296-304.:296-304.
Morais, R., P.Desjardins, C.Turmel, and K.Zinkewich-Peotti. 1988. Development and characterization of continuous avian cell lines depleted of mitochondrial DNA. In Vitro Cell Dev. Biol. 24:649-658.
Morais, R., M.Gregoire, L.Jeannotte, and D.Gravel. 1980. Chick embryo cells rendered respiration-deficient by chloramphenicol and ethidium bromide are auxotrophic for pyrimidines. Biochem Biophys Res Commun 94:71-7.
Nemoto,Y., and De Camilli,P. 1999. Recruitment of an alternatively spliced form of synaptojanin 2 to mitochondria by the interaction with the PDZ domain of a mitochondrial outer membrane protein. EMBO J. 18(11):2991-3006.
Neuspiel, M., R.Zunino, S.Gangaraju, P.Rippstein, and H.McBride. 2005. Activated mitofusin 2 signals mitochondrial fusion, interferes with Bax activation, and reduces susceptibility to radical induced depolarization. J. Biol. Chem. 280:25060-25070.
Pavlakis, S.G., P.C.Phillips, S.DiMauro, V.De, and L.P.Rowland. 1984. Mitochondrial myopathy, encephalopathy, lactic acidosis, and strokelike episodes: a distinctive clinical syndrome. Ann. Neurol. 16:481-488.
Rubin, E. and C.S.Lieber. 1968. Early fine structural changes in the human liver induced by alcohol. Gastroenterology. 54:Suppl-3.
Samorajski, T., J.M.Ordy, and J.R.Keefe. 1966. Structural organization of the retina in the tree shrew (Tupaia glis). J. Cell Biol. 28:489-504.
Suzuki, K. 1969. Giant hepatic mitochondria: production in mice fed with cuprizone. Science. 163:81-82.
Tandler, B. and C.L.Hoppel. 1973. Division of giant mitochondria during recovery from cuprizone intoxication. J. Cell Biol. 56:266-272.
Teranishi, M., M.Karbowski, C.Kurono, T.Soji, and T.Wakabayashi. 1999. Two types of the enlargement of mitochondria related to apoptosis: simple swelling and the formation of megamitochondria. J. Electron Microsc. (Tokyo). 48:637-651.
Varadi, A., L.I.Johnson-Cadwell, V.Cirulli, Y.Yoon, V.J.Allan, and G.A.Rutter. 2004. Cytoplasmic dynein regulates the subcellular distribution of mitochondria by controlling the recruitment of the fission factor dynamin-related protein-1. J. Cell Sci. 117:4389-4400.
Wakabayashi, T. 2002. Megamitochondria formation - physiology and pathology. J. Cell Mol. Med. 6:497-538.
Wakabayashi, T. 1999. Structural changes of mitochondria related to apoptosis: swelling and megamitochondria formation. Acta Biochim. Pol. 46:223-237.
Wakabayashi, T. and M.Karbowski. 2001. Structural changes of mitochondria related to apoptosis. Biol. Signals Recept. 10:26-56.
Wakabayashi, T., K.Yamashita, K.Adachi, K.Kawai, M.Iijima, K.Gekko, T.Tsudzuki, J.Popinigis, and M.Momota. 1987. Changes in physicochemical properties of mitochondrial membranes during the formation process of megamitochondria induced by hydrazine. Toxicol. Appl. Pharmacol. 87:235-248.
Yokoo, H., S.K.Singh, and A.H.Hawasli. 1978. Giant mitochondria in alcoholic liver disease. Arch. Pathol. Lab Med. 102:213-214.
Yoon, Y., K.R.Pitts, S.Dahan, and M.A.McNiven. 1998. A novel dynamin-like protein associates with cytoplasmic vesicles and tubules of the endoplasmic reticulum in mammalian cells. J. Cell Biol. 140:779-793.

## Claims

1. An in-vitro method for the formation of megamitochondria in cells wherein the cells are grown in a suitable fermentation medium acidified with lactic acid to pH values between 5.3 and 6.7.

2. The method of claim 1, wherein the pH value is 5.7 to 6.3.

3. The method of claim 1 or 2, wherein the fermentation medium is Isocove medium, RPMI medium, Dulbecco's MEM medium, MEM medium, F12 Medium, Dulbecco's modified Eagle's medium or Minimum Essential Medium Eagle.

4. The method of any of claims 1 to 3, wherein the concentration of lactic acid contained in the medium is 3 to 300 mM.

5. The method of any of claims 1 to 4, wherein the megamitochondria are reversible by exchanging the fermentation medium which was acidified with lactic acid by a common fermentation medium having pH greater than 7.0.

6. An H⁺ ionophore and/or an ionophore which catalyzes the electroneutral exchange of K⁺ for H⁺ for the prevention or treatment of a disease in which inhibiting or reducing the formation of megamitochondria has a beneficial effect.

7. An inhibitor of actin polymerisation for the prevention or treatment of a disease in which inhibiting or reducing the formation of megamitochondria has a beneficial effect.

8. The ionophore of claim 6, which is CCCP or nigericin.

9. The inhibitor of claim 7, which is cytochalasin B.

10. The ionophore or inhibitor of any one of claims 6 to 9 for the prevention or treatment of mitochondrial myopathy, lactic acidosis or MELAS.

11. Use of an H⁺ ionophore and/or an ionophore which catalyzes the electroneutral exchange of K⁺ for H⁺ for the preparation of a pharmaceutical composition for the prevention or treatment of a disease in which inhibiting or reducing the formation of megamitochondria has a beneficial effect.

12. Use of an inhibitor of actin polymerisation for the preparation of a pharmaceutical composition for the prevention or treatment of a disease in which inhibiting or reducing the formation of megamitochondria has a beneficial effect.

13. Use according to claim 11, wherein said ionophore is CCCP or nigericin.

14. Use according to claim 12, wherein said inhibitor is cytochalasin B.

15. Use according to any one of claims 11 to 14, wherein said disease is mitochondrial myopathy, lactic acidosis or MELAS.
